# EUROPÄISCHE PATENTSCHRIFT

(11) **EP 3 427 727 B1**
(45) Veröffentlichungstag und Bekanntmachung des Hinweises auf die Patenterteilung: **28.12.2022**
(21) Anmeldenummer: 17181566.5
(22) Anmeldetag: 14.07.2017
(51) Int. Cl.: A61K 31/065, A61K 31/122, A61P 17/00

(54) **KOMBINATION VON BIOLOGISCH AKTIVEN SUBSTANZEN ZUR STABILISIERUNG ODER ERHÖHUNG DES KOLLAGENGEHALTS**
COMBINATION OF BIOLOGICALLY ACTIVE SUBSTANCES FOR STABILIZING OR INCREASING THE COLLAGEN CONTENT
COMBINAISON DE SUBSTANCES BIOLOGIQUEMENT ACTIVES PERMETTANT DE STABILISER OU D'AUGMENTER LA TENEUR EN COLLAGÈNE

(43) Veröffentlichungstag der Anmeldung: 16.01.2019
(73) Patentinhaber: BioActive Food GmbH, 23795 Bad Segeberg (DE)
(72) Erfinder: VOLLERT, Henning, 23795 Bad Segeberg (DE)
(74) Vertreter: Uexküll & Stolberg

(56) Entgegenhaltungen:
- EP-A1- 2 561 869
- FR-A1- 2 821 549
- US-A1- 2011 142 766
- GENTILI A ET AL: "Evaluation of a method based on liquid chromatography-diode array detector-tandem mass spectrometry for a rapid and comprehensive characterization of the fat-soluble vitamin and carotenoid profile of selected plant foods", JOURNAL OF CHROMATOGRAPHY A, ELSEVIER, AMSTERDAM, NL, Bd. 1218, Nr. 5, 4. Februar 2011 (2011-02-04), Seiten 684-697, XP027594001, ISSN: 0021-9673, DOI: 10.1016/J.CHROMA.2010.12.001 [gefunden am 2011-01-07]

## Beschreibung

Die vorliegende Erfindung betrifft eine synergistisch wirkende Zusammensetzung, die (a) das Carotinoid Lutein, und (b) Vitamin K oder ein Derivat davon ausgewählt aus der Gruppe bestehend aus Vitamin K1, Vitamin K2 und Vitamin K3 umfasst, zur Verwendung in einem Verfahren zur Behandlung und/oder Prophylaxe von Zuständen oder Erkrankungen die mit einer Verringerung der Kollagen-Menge oder mit einem pathologischen Abbau von Kollagen assoziiert sind. Die Erfindung betrifft ferner die nicht-therapeutische Verwendung einer solchen Zusammensetzung oder eines diätetischens Lebensmittels oder eines Nahrungsergänzungsmittels, das eine solche Zusammensetzung umfasst, zur Stabilisierung oder Erhöhung der Kollagen-Menge in menschlichem oder tierischem Gewebe.

### GEBIET DER ERFINDUNG

Kollagen ist ein wichtiger Bestandteil von verschiedenen Geweben, wie beispielsweise Knochen, Zähnen, Haut, Knorpel, Sehnen, Bändern, Faszien und Gefäßen bei Tier und Mensch. Im menschlichen Körper ist Kollagen mit über 30% Anteil an der Gesamtmasse aller Proteine das am häufigsten vorkommende Protein. Kollagenfasern besitzen eine hohe Zugfestigkeit und sind kaum dehnbar. Kollagen ist nicht nur ein wichtigstes Element, das Struktur und Festigkeit der Gewebe ermöglicht, sondern hat zahlreiche weitere biologische Funktionen. So spielen Kollagene bei der Biomineralisation der Wirbeltiere eine entscheidende Rolle (Ehrlich H., International Geology Review, Band 52, Nr. 7-8, 30, 2010, Seiten 661-699). Bestimmte im Auge vorkommende Kollagene sind transparent und ermöglichen die hohe Brechkraft des Auges.

Seit kurzem ist es möglich, die Menge des Kollagens in der Haut von lebenden Tieren oder Menschen nicht-invasiv zu quantifizieren und die Veränderung der Kollagen-Menge über längere Zeiträume zu messen. Hierbei werden zwei Verfahren kombiniert: Einerseits ein nicht-invasives, hochaufgelöstes Bildgebungsverfahren, das auf einer sog. Multiphotonen-Mikroskopie basiert, und andererseits die Messung eines sogenannten "Second Harmonic Generation"-Signals (SHG), bei dem Kollagen das elektrische Feld des eingestrahlten Lichtes verändert (Pittet et al., Cosmetics 2014, 1, 211-221). Durch das Verhältnis zwischen dem SHG und dem Autofluoreszenz-Signal des Elastins können Kollagen und Elastin in der Haut quantifiziert und in ein Verhältnis gesetzt (sog. SHG-AF-Index der Dermis - SAID) werden.

Die Menge des Kollagens nimmt beim Menschen ab etwa dem 25. Lebensjahr ab. Dies liegt möglicherweise daran, dass Kollagen nicht mehr in ausreichend hohem Maße gebildet wird und stärker durch die Aktivität von verschiedenen Enzymen, wie z.B. zu den Matrix-Metalloproteasen zählenden Kollagenasen, abgebaut wird. Kollegenasen werden u.a. von Menschen und Bakterien gebildet. Die Expression dieser Kollagenasen kann wiederum durch verschiedene Parameter verändert werden. Die physiologischen Prozesse, die die Kollagen-Menge beeinflussen, sind komplex. Mit steigendem Alter aber auch durch Umwelteinflüsse, erhöhte Blutglucose-Spiegel oder Tumorpromotoren nehmen beispielsweise die Expression und die Aktivität von Proteinkinase-C in Hautfibroblasten zu. Diese wiederum steuert die Expression der Kollagenase MMP-1. MMP-1 kann Kollagen enzymatisch spalten und auf diese Weise abbauen. Faktoren, die die Blutglucose, Expression und Aktivität der Proteinkinase-C beeinflussen, haben daher einen indirekten Einfluss auf die Kollagen-Menge.

Es gibt zahlreiche Erkrankungen, bei denen eine erhöhte Aktivität der Kollagenasen belegt wurde (z.B. Fibrosen). Des Weiteren kann die Einnahme von verschiedenen Medikamenten (beispielsweise Cortison und Chemotherapeutika) zu einer Verringerung der Kollagen-Menge führen. Es gibt Hinweise, dass beispielsweise Übergewicht und Diabetes einen Einfluss auf die Kollagen-Menge haben (Haluszka et al., Biomed Opt Express. 2016; 7(11):4480-4489). Auch bei Krebs können sich Menge und Zusammensetzung der Kollagene verändern (Golaraei et al., Biomed Opt Express. 2016; 15; 7(10):4054-4068). Die Mechanismen, welche hierbei die Kollagen-Menge beeinflussen, sind erst teilweise aufgeklärt.

Autoimmunerkrankungen die zu Kollagenosen führen, können behandelt werden, indem die Aktivität des Immunsystems durch Medikamente verringert wird. Neben nicht-steroidalen Antiphlogistika wie Diclofenac oder Ibuprofen werden D-Penicillamin (vor allem bei der Sklerodermie), Chloroquin (bei Lupus und Sjögren-Syndrom), Azathioprin, Methotrexat, weitere moderne Immunsuppressiva und Kortikoide (bei allen Kollagenosen) eingesetzt. Allerdings ist die Behandlung mit Immunsuppressiva und Kortikoiden aufgrund der beachtlichen Nebenwirkungen nicht problemfrei.

Es konnte eine kollagenerhöhende Wirkung mittels des Naturstoffs Menachinon-7 aus der Gruppe der K-Vitamine (EP 2 561 869 A1) oder mittels Substanzen aus der Gruppe der Carotinoide (FR 2821549 A1) festgestellt werden.

Aufgabe der vorliegenden Erfindung ist es, neue Zusammensetzungen und Verfahren bereitzustellen, mithilfe derer die Kollagen-Menge in tierischen und menschlichen Geweben stabilisiert und/oder erhöht werden kann und/oder das Ausmaß des Abbaus von Kollagen in diesen Geweben verringert werden kann und die darüber hinaus minimale oder keine Nebenwirkungen aufweisen. Erfindungsgemäß wurde nunmehr herausgefunden, dass diese Ziele durch eine Kombination von Lutein und Vitamin K bzw. den Vitamin K-Derivaten Vitamin K1, Vitamin K2 und Vitamin K3 erreicht werden kann. Die erfindungsgemäßen Zusammensetzungen erweisen sich als hochwirksam und praktisch nebenwirkungsfrei.

### BESCHREIBUNG DER ERFINDUNG

Demgemäß betrifft die Erfindung in einem ersten Aspekt Zusammensetzungen, die mindestens die folgenden beiden Bestandteile enthalten: (a) mindestens das Carotinoid Lutein und (b) Vitamin K oder ein Derivat davon ausgewählt aus der Gruppe bestehend aus Vitamin K1, Vitamin K2 und Vitamin K3. Die erfindungsgemäßen Zusammensetzungen werden erfindungsgemäß zur Verwendung in einem Verfahren zur Behandlung und/oder Prophylaxe von Zuständen oder Erkrankungen, die mit einer Verringerung der Kollagen-Menge oder mit einem pathologischen Abbau von Kollagen assoziiert sind, vorgeschlagen. Die Zusammensetzungen zeichnen sich durch eine synergistische Wirkung ihrer Bestandteile aus, d.h. das Zusammenwirken der einzelnen Bestandteile geht über einen rein additiven Effekt hinaus.

Wie oben dargelegt umfassen die erfindungsgemäßen Zusammensetzungen mindestens ein Carotinoid, wobei es sich bei dem Carotinoid um Lutein handelt. Als Carotinoide werden vorliegend eine Gruppe natürlich vorkommender, fettlöslicher Pigmente bezeichnet, denen chemisch als Grundgerüst ein Tetraterpen gemeinsam ist. Tier und Mensch können Carotinoide nicht synthetisieren. Sie werden über die Nahrung aufgenommen. In Leber, Augen, Gehirn, Haut und Fettgewebe liegen bestimmte Carotinoide in deutlich höherer Konzentration vor als in anderen Körpergeweben. In der Netzhaut des Auges, im sogenannten gelben Fleck (Macula), kommen die Carotinoide Lutein und Zeaxanthin in größeren Mengen vor. Diese Carotinoide wirken hier als natürliche Schutzmechanismen, da die Netzhaut mit ihren besonders oxidationsempfindlichen mehrfach ungesättigten Fettsäuren besonders anfällig für den Angriff von freien Radikalen ist.

Die derzeit bekannten Carotinoide werden in Carotine einerseits und sauerstoffhaltige Xanthophylle andererseits unterschieden. Carotine stellen eine Gruppe von 11- bis 12-fach ungesättigten Triterpenen dar. Von besonderer Bedeutung sind die drei isomeren α-, β- und γ-Carotine, die alle über das gleiche Grundgerüst mit 9 konjugierten Doppelbindungen, 8 Methylverzweigungen einschließlich möglicher Ringstrukturen und einer β-Ionon-Ringstruktur an einem Molekülende verfügen.

Nachstehend sind beispielhaft Carotine gezeigt, die neben Lutein als zusätzlicher Bestandteil der erfindungsgemäßen Zusammensetzungen verwendet werden können. Gemäß einer bevorzugten Ausführungsform der Erfindung handelt es sich bei dem zusätzlichen Carotin, das in der erfindungsgemäßen Zusammensetzung enthalten ist, um α-Carotin. Gemäß einer weiteren bevorzugten Ausführungsform der Erfindung handelt es sich bei dem zusätzlichen Carotin um β-Carotin. Gemäß einer noch weiteren bevorzugten Ausführungsform der Erfindung handelt es sich bei dem zusätzlichen Carotin um γ-Carotin. Gemäß einer noch weiteren bevorzugten Ausführungsform der Erfindung handelt es sich bei dem zusätzlichen Carotin um δ-Carotin. Gemäß einer noch weiteren bevorzugten Ausführungsform der Erfindung handelt es sich bei dem zusätzlichen Carotin um ε-Carotin. Gemäß einer noch weiteren bevorzugten Ausführungsform der Erfindung handelt es sich bei dem zusätzlichen Carotin um Lycopen.

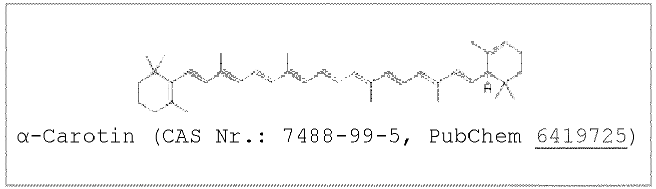

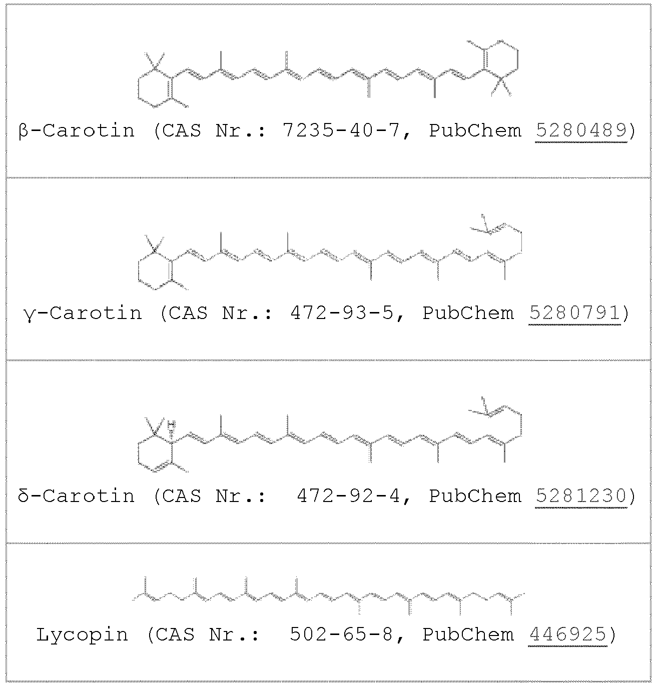

Die erfindungsgemäße Zusammensetzung umfasst das Carotinoid Lutein aus der Gruppe der Xanthophylle. Im Gegensatz zu Carotinen enthalten Xanthophylle Sauerstoff. Das Grundgerüst besteht aus 8 Isopren-Einheiten (Tetraterpene). Die Xanthophylle sind aus zwei C20-Isoprenopiden derart zusammengesetzt, dass die beiden mittleren Methylgruppen in 1,6-Stellung zueinander stehen. Bevorzugte Xanthophylle, die im Rahmen der vorliegenden Erfindung Verwendung finden können, umfassen:
- (3R,6'R)-β-ε-Carotin-3,3'-diol (Lutein),
- (3R,3'S,5'R)-3,3'-Dihydroxy-β,κ-carotin-6-on (Capsanthin),
- 9'-cis-6,6'-Diapocarotindisäure-6'-methylester (Bixin),
- (3S,3'S,5R,5'R)-3,3'-Dihydroxy-κ,κ-carot-in-6,6'-dion (Capsorubin), und
- (3S,3'S)-3,3'-Dihydroxy-β,β'-carotin-4,4'-dion (Astaxanthin).

Gemäß einer bevorzugten Ausführungsform der Erfindung umfasst die erfindungsgemäße Zusammensetzung neben Lutein noch weitere Xanthophylle. Gemäß einer bevorzugten Ausführungsform der Erfindung handelt es sich bei dem zusätzlichen Xanthophyll um Capsanthin. Gemäß einer noch weiteren bevorzugten Ausführungsform der Erfindung handelt es sich bei dem zusätzlichen Xanthophyll um Bixin. Gemäß einer noch weiteren bevorzugten Ausführungsform der Erfindung handelt es sich bei dem zusätzlichen Xanthophyll um Capsorubin. Gemäß einer noch weiteren bevorzugten Ausführungsform der Erfindung handelt es sich bei dem zusätzlichen Xanthophyll um Astaxanthin.

Neben den Carotinen und Xanthophyllen können erfindungsgemäß auch deren Spaltprodukte, wie beispielsweise 3,7-Dimethyl-9-(2,6,6-trimethyl-1-cyclohexenyl)-2,4,6,8-nonatetraen-1-ol (Retinol, Vitamin A1) und 3,7-Dimethyl-9-(2,6,6-trimethyl-1-cyclohexenyl)-2,4,6,8-nonatetraenal (Retinal, Vitamin A1-Aldehyd) verwendet werden.

Als weiteren Bestandteil umfassen die erfindungsgemäßen Zusammensetzungen Vitamin K oder ein Derivat davon ausgewählt aus der Gruppe bestehend aus Vitamin K1, Vitamin K2 und Vitamin K3. K-Vitamine gehören neben den Vitaminen A, D und E zu den fettlöslichen Vitaminen. Sie fungieren als Cofaktor in Reaktionen der γ-Glutamylcarboxylase. Über diesen Mechanismus werden mehrere Gerinnungsfaktoren in einen aktivierbaren Zustand versetzt und gerinnungshemmende Faktoren aktiviert und reguliert. Ferner spielt Vitamin K für die Aktivierung von mehreren Vitamin K-abhängigen Proteinen, wie beispielsweise Osteocalcin oder Matrix-Gla-Protein, eine wichtige Rolle beim Aufbau und bei der Stabilität von Knochen und Knorpel. Über die Nahrung nimmt der Mensch vor allem das in Pflanzen vorkommende Vitamin K1 und das von Bakterien gebildete Vitamin K2 auf. Das aufgenommene Vitamin K1 wird durch die Mitochondrien der menschlichen Zellen in Vitamin K2 umgewandelt (Vos M. et al.). Dort fungiert es u.a. als Elektronen-Carrier in den Mitochondrien.

Vitamin K1 kommt in den Chloroplasten der Blätter von verschiedenen Gemüsesorten, wie z.B. Kohl, Brennnesseln, Luzernen, Spinat wie auch in Früchten (z.B. Tomaten, Erdbeeren, Hagebutten) sowie in Muskelfleisch, Leber, Milch und Eiern vor. Der Tagesbedarf liegt bei etwa 1 bis 2 µg/kg Körpergewicht beim Erwachsenen und älteren Kind, beim Säugling bei etwa 10 bis 20 µg/kg. Über die Wirkung von Vitamin K auf die Haut ist wenig bekannt. So haben Li et al. eine Creme mit Vitamin K getestet, die den Juckreiz mildert. Auch eine Wirkung von Vitamin K gegen Rötungen und Einblutungen bei Laserbehandlungen wurde beschrieben (Lou et al.). Neben den natürlichen Vitaminen K1 und K2 können auch synthetische Derivate von Vitamin K verwendet werden, wie beispielsweise Vitamin K3. Die Vitamine K1, K2 und K3 sind nachstehend gezeigt.

| | |
|---|---|
| 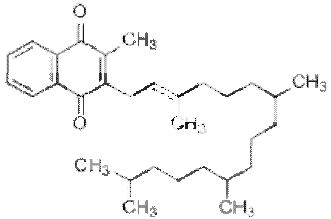 | Vitamin K1, CAS-Nr. : 84-80-0 |
| 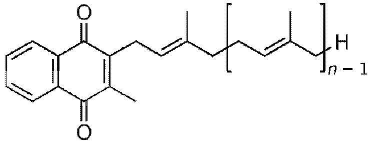 | Vitamin K 2, CAS-Nr.: 84-81-1 |
| 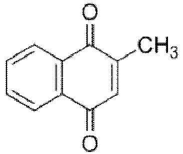 | Vitamin K 3, CAS-Nr.: 58-27-5 |

Die synergistisch wirkende Zusammensetzung der Erfindung kann mehr als ein Carotinoid enthalten, wie beispielsweise 1, 2, 3, 4, 5 oder mehr Carotinoide. Ebenso kann die synergistisch wirkende Zusammensetzung der Erfindung mehr als ein Vitamin K enthalten, wie beispielsweise Vitamin K1 und K2, Vitamin K1 und K3, Vitamin K2 und K3, oder Vitamin K1, K2 und K3.

Der Fachmann ist problemlos in der Lage, die für die jeweils zu erreichende (therapeutische) Wirkung erforderliche Menge an Vitamin K und Carotinoid durch einfache Versuchsreihen zu ermitteln. Hierbei wird der Fachmann verschiedene Faktoren berücksichtigen, wie z.B. die Art und Menge des oder der in der Zusammensetzung vorhandenen Carotinoide, die Art und Menge der zusätzlich in den Zusammensetzungen vorhandenen Komponenten, Alter und Gewicht der Person, an die die Verabreichung der Zusammensetzung erfolgt, usw.

Die Menge an Carotinoid in den erfindungsgemäßen Zusammensetzungen ist vorzugsweise größer als 1 ppm (parts per million) oder 0,000001 % (w/w). Es ist bevorzugt, dass die Menge an Carotinoid in der Zusammensetzung größer ist als 10 ppm, größer als 100 ppm, größer als 1000 ppm, größer als 10⁴ ppm, größer als 5×10⁴ ppm oder größer als 10⁵ ppm.

Anders ausgedrückt enthält die erfindungsgemäße Zusammensetzung mehr als 0,0001% (w/w) mehr als 0,001%, mehr als 0,01%, mehr als 0,1%, mehr als 1%, mehr als 2%, mehr als 3%, mehr als 4%, mehr als 5%, mehr als 6%, mehr als 7%, mehr als 8%, mehr als 9%, mehr als 10%, mehr als 11%, mehr als 12%, mehr als 13%, mehr als 14% oder mehr als 15% an Carotinoid, wie z.B. Lutein. Ebenfalls bevorzugt ist es, dass die erfindungsgemäße Zusammensetzung mehr als 20% (w/w), mehr als 25%, mehr als 30%, mehr als 35%, mehr als 40%, mehr als 45%, mehr als 50%, mehr als 55% oder mehr als 60% des Carotinoids, wie z.B. Lutein, umfasst.

In einer besonders bevorzugten Ausführungsform enthalten die erfindungsgemäßen Zusammensetzungen zwischen 1-95% (w/w), wie z.B. 5-80%, 10-70%, 15-60%, 20-50%, oder 30-40% an Carotinoid, wie z.B. Lutein, z.B. 5-95%, 10-95%, 15-95%, 20-95%, oder 30-95% an Carotinoid, wie z.B. Lutein. In einer bevorzugten Ausführungsform beträgt die Menge an Carotinoid, wie z.B. Lutein, etwa 0,1%, 1%, 2%, 3%, 4%, 5%, 10%, 20%, 30%, 40%, 50%, 60%, 70%, 80%, 90%, oder 95%.

Die Menge an Carotinoid in den erfindungsgemäßen Zusammensetzungen liegt vorzugsweise zwischen 0,0001 und 1000 mg, z.B. zwischen 0,0001 und 100 mg, zwischen 0,01 und 10 mg, oder zwischen 0,1 und 10 mg.

Die erfindungsgemäße Zusammensetzung wird vorzugsweise so formuliert, dass die jeweilige Verabreichungseinheit (z.B. eine Kapsel, eine Tablette oder eine definierte Menge einer Flüssigkeit) eine Menge von 0,0001 bis 1 g an Carotinoid, wie z.B. Lutein, enthält, z.B. mehr als 0,001 g, mehr als 0,01 g, mehr als 0,02 g, mehr als 0,03 g, mehr als 0,04 g, mehr als 0,05 g, mehr als 0,1 g, mehr als 0,5 g, oder mehr als 1 g.

In einer Ausführungsform handelt es sich bei dem Carotinoid, der in den obigen Mengen in der erfindungsgemäßen Zusammensetzung vorliegt, um ein Xanthophyll. In einer weiteren Ausführungsform handelt es sich bei dem Carotinoid, der in den obigen Mengen in der erfindungsgemäßen Zusammensetzung vorliegt, um Lutein.

Die Menge an Vitamin K oder an Vitamin K-Derivat in den erfindungsgemäßen Zusammensetzungen ist vorzugsweise ebenfalls größer als 1 ppm (parts per million) oder 0,000001% (w/w). Es ist bevorzugt, dass die Menge an einem Vitamin K oder Vitamin K-Derivat in der Zusammensetzung größer ist als 10 ppm, größer als 100 ppm, größer als 1000 ppm, größer als 10⁴ ppm, größer als 5×10⁴ ppm oder größer als 10⁵ ppm.

Anders ausgedrückt enthält die erfindungsgemäße Zusammensetzung mehr als 0,0001% (w/w), mehr als 0,001%, mehr als 0,01%, mehr als 0,1%, mehr als 1%, mehr als 2%, mehr als 3%, mehr als 4%, mehr als 5%, mehr als 6%, mehr als 7%, mehr als 8%, mehr als 9%, mehr als 10%, mehr als 11%, mehr als 12%, mehr als 13%, mehr als 14% oder mehr als 15% Vitamin K oder Vitamin K-Derivat. Ebenfalls bevorzugt ist es, dass die erfindungsgemäße Zusammensetzung mehr als 20% (w/w), mehr als 25%, mehr als 30%, mehr als 35%, mehr als 40%, mehr als 45%, mehr als 50%, mehr als 55%, oder mehr als 60% Vitamin K oder Vitamin K-Derivat umfasst.

In einer besonders bevorzugten Ausführungsform enthalten die erfindungsgemäßen Zusammensetzungen zwischen 1-95% (w/w), wie z.B. 5-80%, 10-70%, 15-60%, 20-50% oder 30-40% Vitamin K oder Vitamin K-Derivat, oder wie z.B. 5-95%, 10-95%, 15-95%, 20-95%, oder 30-95% Vitamin K oder Vitamin K-Derivat. In einer bevorzugten Ausführungsform beträgt die Menge an Vitamin K, wie z.B. Vitamin K1, etwa 0,1%, 1%, 2%, 3%, 4%, 5%, 10%, 20%, 30%, 40%, 50%, 60%, 70%, 80%, 90%, oder 95%.

Die Menge an Carotinoid in den erfindungsgemäßen Zusammensetzungen liegt vorzugsweise zwischen 0,0001 und 250 mg, z.B. zwischen 0,0001 und 25 mg, zwischen 0,01 und 2,5 mg, oder zwischen 0,1 und 0,25 mg.

Die erfindungsgemäße Zusammensetzung wird vorzugsweise so formuliert, dass die jeweilige Verabreichungseinheit (z.B. eine Kapsel, eine Tablette oder eine definierte Menge einer Flüssigkeit) eine Menge von 0,0001 bis 1 g Vitamin K, wie z.B. Vitamin K1, enthält, z.B. mehr als 0,001 g, mehr als 0,01 g, mehr als 0,02 g, mehr als 0,03 g, mehr als 0,04 g, mehr als 0,05 g, mehr als 0,1 g, mehr als 0,5 g, oder mehr als 1 g.

In einer Ausführungsform handelt es sich bei dem Vitamin K, das in den obigen Mengen in der erfindungsgemäßen Zusammensetzung vorliegt, um Vitamin K1. In einer weiteren Ausführungsform handelt es sich bei dem Vitamin K, das in den obigen Mengen in der erfindungsgemäßen Zusammensetzung vorliegt, um Vitamin K2. In einer noch weiteren Ausführungsform handelt es sich bei dem Vitamin K, das in den obigen Mengen in der erfindungsgemäßen Zusammensetzung vorliegt, um Vitamin K3.

Das Einsatzverhältnis zwischen den Komponenten (a) und (b) der erfindungsgemäßen Zusammensetzung kann bezogen auf die Gewichtsteile 499:1 bis 1:499, vorzugsweise 75:1 bis 1:75 und insbesondere 50:1 bis 1:50 betragen. Besonders bevorzugt ist es, dass der Carotinoid-Bestandteil und der Vitamin K-Bestandteil in etwa gleichen Mengen vorliegen.

Besonders bevorzugt ist es, dass die erfindungsgemäße Zusammensetzung für die orale Verabreichung formuliert ist und von 0,001 bis 25 mg Vitamin K oder Vitamin K-Derivat, vorzugsweise von 0,01 mg bis 10 mg Vitamin K oder Vitamin K-Derivat enthält sowie von 0,001 bis 25 mg Carotinoid, vorzugsweise von 0,01 bis 10 mg Carotinoid. In einer weiteren Ausführungsform der Erfindung beträgt die Menge an Carotinoid in der Zusammensetzung mindestens 0,01 g, und die Menge eines Vitamin K in der Zusammensetzung beträgt ebenfalls mindestens 0,01 g. In einer noch weiteren Ausführungsform beträgt die Menge an Carotinoid in der Zusammensetzung zwischen 0,1 und 1 g, und die Menge an Vitamin K oder Vitamin K-Derivat in der Zusammensetzung beträgt zwischen 0,1 und 1 g.

Die Zusammensetzungen der vorliegenden Erfindung eignen sich insbesondere zur Verwendung in einem Verfahren zur Stabilisierung oder Erhöhung der Kollagen-Menge in menschlichem oder tierischem Gewebe. Somit eignen sich die Zusammensetzung der vorliegenden Erfindung insbesondere zur Behandlung und/oder Prophylaxe von Zuständen oder Erkrankungen die mit einer Verringerung der Kollagen-Menge oder mit einem pathologischen Abbau von Kollagen assoziiert sind, insbesondere in Geweben wie Haut, Knorpel oder Knochen. Die Zusammensetzungen können ferner zur Milderung von Nebenwirkungen von Medikamenten, wie z.B. Kortisol-Steroiden eingesetzt werden. Besondere Anwendung finden die Zusammensetzungen der vorliegenden Erfindung bei der Behandlung von Kollagenosen, wie z.B. systemischer Lupus erythematodes (SLE), Polymyositis und Dermatomyositis, Sjögren-Syndrom, Sklerodermie, CREST-Syndrom, Sharp-Syndrom, Buschke-Ollendorf-Syndrom und systemischer Sklerose.

Die Erfindung offenbart ferner die Verwendung einer wie oben beschriebenen Zusammensetzung zur Herstellung einer pharmazeutischen Zusammensetzung, eines diätetischen Lebensmittels und/oder eines Nahrungsergänzungsmittels.

In einem noch weiteren Aspekt betrifft die vorliegende Erfindung somit auch eine pharmazeutische Zusammensetzung, ein diätetisches Lebensmittel oder ein Nahrungsergänzungsmittel, die/das eine wie oben beschriebene Zusammensetzung umfasst, welche mindestens Lutein und Vitamin K oder ein Vitamin K-Derivat ausgewählt aus der Gruppe bestehend aus Vitamin K1, Vitamin K2 und Vitamin K3 umfasst. Die pharmazeutische Zusammensetzung, das diätetische Lebensmittel oder das Nahrungsergänzungsmittel eignen sich ebenfalls für die oben genannten Zwecke, wie beispielsweise zur Behandlung und/oder Prophylaxe von Zuständen oder Erkrankungen die mit einer Verringerung der Kollagen-Menge oder mit einem pathologischen Abbau von Kollagen assoziiert sind.

Eine die erfindungsgemäße Zusammensetzung umfassende pharmazeutische Zusammensetzung kann für die orale, parenterale oder topische Darreichung formuliert sein. Eine solche pharmazeutische Zusammensetzung kann mittels im Stand der Technik hinreichend bekannter Verfahren hergestellt werden. Derartige Verfahren sowie geeignete Hilfsstoffe und Träger werden beispielsweise in "Remington: The Science and Practice of Pharmacy", Lippincott Williams & Wilkins; 21. Auflage (2005) beschrieben. Die pharmazeutischen Zusammensetzungen können beispielsweise in Form von Granulaten, Pulvern, Tabletten, Kapseln, Sirup, Suppositorien, Injektionen, Emulsionen, Suspensionen oder Lösungen vorliegen. Die Zusammensetzungen können für verschiedene Verabreichungsarten formuliert sein, beispielsweise für die orale, parenterale, topische, bukkale, sublinguale, transmukosale, rektale, subkutane, intrathekale, intravenöse, intramuskuläre, intraperitoneale, nasale, intraokulare oder intraventrikuläre Verabreichung. Die Formulierung als orale, parenterale oder topische Zusammensetzung ist besonders bevorzugt. In einer besonders bevorzugten Ausführungsform ist die Zusammensetzung für die orale Verabreichung formuliert. Die pharmazeutischen Zusammensetzungen können auch als Retardmittel formuliert werden.

Für die orale, bukkale und sublinguale Verabreichung werden in der Regel feste Formulierungen wie, z.B. Pulver, Suspensionen, Granulate, Tabletten, Pillen, Kapseln und Gelcaps verwendet. Diese können beispielsweise hergestellt werden, indem man die wirksamen Bestandteile (Carotinoid und Vitamin K) mit mindestens einem Additiv oder mit mindestens einem Hilfsstoff vermischt. Derartige Hilfsstoffe und Träger werden beispielsweise in "Remington: The Science and Practice of Pharmacy", Lippincott Williams & Wilkins; 21. Auflage (2005) beschrieben. Als Additive oder Hilfsstoffe können beispielsweise mikrokristalline Cellulose, Methylcellulose, Hydroxypropylmethylcellulose, Casein, Albumin, Mannit, Dextran, Saccharose, Lactose, Sorbitol, Stärke, Agar, Alginate, Pectine, Kollagen, Glyceride oder Gelatine verwendet werden. Ferner können Zusammensetzungen für die orale Verabreichung Antioxidantien (z.B. Ascorbinsäure, Tocopherol oder Cystein), Gleitmittel (z.B. Magnesiumstearat), Konservierungsmittel (z.B. Paraben oder Sorbinsäure), Geschmacksverbesserer, Sprengmittel, Bindemittel, Verdicker, Farbstoffe und ähnliche Substanzen umfassen.

Flüssige Formulierungen der erfindungsgemäßen Zusammensetzungen, die sich für die orale Verabreichung eignen, können beispielsweise als Emulsion, Sirup, Suspension oder Lösungen vorliegen. Diese Formulierungen können unter Verwendung einer sterilen Flüssigkeit als Träger (z.B. Öl, Wasser, Alkohol oder Kombinationen derselben) in Form von flüssigen Suspensionen oder Lösungen hergestellt werden. Für die orale oder parenterale Verabreichung können pharmazeutisch geeignete Tenside, Suspensionsmittel, Öle oder Emulgatoren zugefügt werden. Für den Gebrauch in flüssigen Dosisformen geeignete Öle umfassen beispielsweise Olivenöl, Sesamöl, Erdnussöl, Rapsöl und Maisöl. Geeignete Alkohole umfassen Ethanol, Isopropylalkohol, Hexadecylalkohol, Glycerin und Propylenglycol. Suspensionen können ferner Fettsäureester, wie Ethyloleat, Isopropylmyristat, Fettsäureglyceride und acetylierte Fettsäureglyceride umfassen. Ferner werden Suspensionen häufig auch mit Substanzen wie Mineralöl oder Petrolatum versetzt.

### FIGUREN

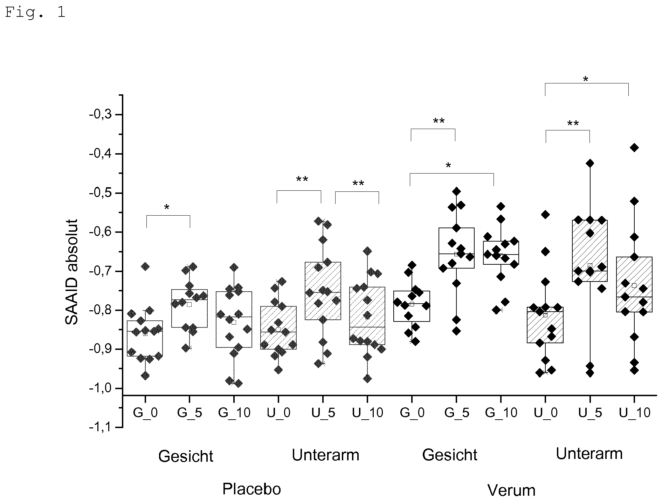

Fig. 1 zeigt die Ergebnisse der Messung der Kollagen-Mengen am Unterarm sowie im Gesicht.

### BEISPIELE

Die nachfolgenden Beispiele beschreiben die Wirkung der erfindungsgemäßen Zusammensetzungen:

### Beispiel 1: In vitro Experiment mit Vitamin K und Lutein

In einem zellulären Test wurde die Wirkung von Vitamin K1 und Lutein auf die Kollagen-Synthese untersucht. Die Untersuchung erfolgte nach dem in Suh et al. (Biol Pharm Bull. 2009 Apr; 32(4):746-9) beschriebenen Verfahren. Das gebildete Kollagen wurde mit Hilfe eines Standardassays (Firma Biocolor Ltd., Carrickfergus, Großbritannien) bestimmt. Der Assay war in der Lage, Kollagen des Typs I-IV zu erfassen.

### Ergebnisse:

Wie der nachstehenden Tabelle 1 zu entnehmen ist, führte die Verabreichung der Substanzen Kaempferol und Lutein zu einer Erhöhung der Kollagen-Synthese.

| | | Kollagen (relativ) |
|---|---|---|
| 1. | Kontrolle | 100 % |
| 2. | Lutein (2 µM) | 105 % |
| 3. | Vitamin K (0,5 µM) | 116 % |
| 4. | Lutein (1 µM) und Vitamin K (0,25 µM) | 145 % |

Es lässt sich erkennen dass die Erhöhung durch Lutein bzw. Vitamin K 5% bzw. 16% betrug. Die gemeinsame Verabreichung der beiden Substanzen in der jeweils halben Wirkstoffkonzentration hingegen führte zu einer deutlich stärkeren Bildung von Kollagen (45%). Es liegt eine deutliche überadditive, synergistische Wirkung vor.

### Beispiel 2: Humanstudie mit Lutein und Vitamin K1

Die Wirkung der Kombination aus Carotinoid und Vitamin-K wurde von unabhängiger Seite durch das Institut für Dermatologie in Berlin getestet. Die Probanden erhielten 1 ml (3 Kapseln) eines Pflanzenöls, das Lutein (1,5 mg) und Vitamin K1 (20 µg) enthielt. Die Probanden der Placebogruppe erhielten lediglich Olivenöl (1 ml, 3 Kapseln). Der Test verlief über einen Zeitraum von 10 Monaten. Die Menge des Kollagens wurde nicht-invasiv mit Hilfe eines Bildgebungsverfahrens (SAID, s.o.) bestimmt.

Gemessen wurde hierbei an einer definierten Hautstelle im Gesicht. In der Placebogruppe (n=13) erhöhte sich das Kollagen-Elastin-Index nicht signifikant um etwa 0,04 Einheiten. In der Verumgruppe (n=12) stieg der Kollagen-Elastin-Index signifikant um etwa 0,12 Einheiten (p < 0,05) und auch im Vergleich zur Differenz der Placebowerte statistisch signifikant um etwa 0,12 Einheiten (p< 0,005).

| | SAID-Werte (absolut) |
|---|---|
| Placebo, Startwert | -0,86 |
| Placebo, 10 Monate | -0,82 |
| Verum, Startwert | -0,815 |
| Verum, 10 Monate | -0,66 |

Es lässt sich daher feststellen, dass die erfindungsgemäßen Zusammensetzungen bei der Erhöhung der Kollagen-Menge in der menschlichen Haut wirksam sind. Die Ergebnisse sind in Fig. 1 dargestellt.

### LITERATUR

1. Ehrlich: Chitin and collagen as universal and alternative templates in biomineralization. In: International Geology Review. Band 52, Nr. 7-8, 30. April 2010, S. 661-699,
2. Pittet et al., Cosmetics 2014, 1, 211-221,
3. Haluszka et al. Biomed Opt Express. 2016;7(11):4480-4489,
4. Golaraei et al. Biomed Opt Express. 2016; 15;7(10):4054-4068,
5. Shim JS et al. Planta Med. 2008 Feb;74(3):239-44,
6. Cheng et al. Nature Protocols 7, 654-669 (2012),
7. Tsai et al. Biomed Opt Express. 2012 Sep 1; 3(9): 2234-2243,
8. Widomska et al., J Clin Exp Ophthalmol. 2014;5(1):326,
9. Lim et al., Planta Med. 2007;73(12):1267-74,
10. Vos M. et al., Science. 2012 8; 336(6086):1306-10),
11. Li et al., Pak. J. Pharm. Sci., Vol. 28, No. 4, 2015, S. 1499-1503,
12. Lou et al., Dermatol Surg. 1999; 25(12):942-4.

## Patentansprüche

1. Zusammensetzung, umfassend:
(a) mindestens ein Carotinoid, wobei es sich bei dem Carotinoid um Lutein handelt, und
(b) mindestens ein Vitamin K oder Vitamin K-Derivat ausgewählt aus der Gruppe bestehend aus Vitamin K1, Vitamin K2 und Vitamin K3,
zur Verwendung in einem Verfahren zur Behandlung und/oder Prophylaxe von Zuständen oder Erkrankungen die mit einer Verringerung der Kollagen-Menge oder mit einem pathologischen Abbau von Kollagen assoziiert sind.

2. Zusammensetzung zur Verwendung in einem Verfahren nach Anspruch 1, wobei es sich bei dem Vitamin K um Vitamin K1 handelt.

3. Zusammensetzung zur Verwendung in einem Verfahren nach Anspruch 1, wobei es sich bei dem Vitamin K um Vitamin K2 handelt.

4. Zusammensetzung zur Verwendung in einem Verfahren nach Anspruch 1, wobei es sich bei dem Vitamin K-Derivat um Vitamin K3 handelt.

5. Zusammensetzung zur Verwendung in einem Verfahren nach einem der Ansprüche 1-4, wobei die Zusammensetzung von 0,0001 bis 100 mg Carotinoid umfasst.

6. Zusammensetzung zur Verwendung in einem Verfahren nach einem der Ansprüche 1-5, wobei die Zusammensetzung von 0,0001 bis 25 mg Vitamin K oder Vitamin K-Derivat, ausgewählt aus der Gruppe bestehend aus Vitamin K1, Vitamin K2 und Vitamin K3, umfasst.

7. Zusammensetzung zur Verwendung in einem Verfahren nach einem der Ansprüche 1-6, wobei es sich bei den Erkrankungen um Kollagenosen, wie z.B. systemischer Lupus erythematodes (SLE), Polymyositis und Dermatomyositis, Sjögren-Syndrom, Sklerodermie, CREST-Syndrom, Sharp-Syndrom, Buschke-Ollendorf-Syndrom oder systemischer Sklerose handelt.

8. Zusammensetzung zur Verwendung in einem Verfahren nach einem der Ansprüche 1-7, wobei die Zusammensetzung für die orale, parenterale oder topische Darreichung formuliert ist.

9. Pharmazeutische Zusammensetzung, die eine Zusammensetzung nach einem der Ansprüche 1-7 umfasst, zur Verwendung in einem Verfahren nach einem der Ansprüche 1-7.

10. Nicht-therapeutische Verwendung einer Zusammensetzung umfassend:
(a) mindestens ein Carotinoid, wobei es sich bei dem Carotinoid um Lutein handelt, und
(b) mindestens ein Vitamin K oder Vitamin K-Derivat ausgewählt aus der Gruppe bestehend aus Vitamin K1, Vitamin K2 und Vitamin K3,
oder eines diätetischen Lebensmittels oder eines Nahrungsergänzungsmittels, das eine solche Zusammensetzung umfasst, zur Stabilisierung oder Erhöhung der Kollagen-Menge in menschlichem oder tierischem Gewebe.

11. Nicht-therapeutische Verwendung nach Anspruch 10, wobei es sich bei dem menschlichen oder tierischen Gewebe um Haut, Knorpel oder Knochen handelt.

12. Nicht-therapeutische Verwendung nach Anspruch 10 oder 11, wobei die Zusammensetzung für die orale, parenterale oder topische Darreichung formuliert ist.

## Claims

1. Composition comprising:
(a) at least one carotenoid, wherein the carotenoid is lutein, and
(b) at least one vitamin K or vitamin K derivative selected from the group consisting of vitamin K1, vitamin K2 and vitamin K3,
for use in a method of treatment and/or prophylaxis of conditions or diseases which are associated with a reduction in the amount of collagen or with a pathological degradation of collagen.

2. Composition for use in a method according to claim 1, wherein the vitamin K is vitamin K1.

3. Composition for use in a method according to claim 1, wherein the vitamin K is vitamin K2.

4. Composition for use in a method according to claim 1, wherein the vitamin K derivative is vitamin K3.

5. Composition for use in a method according to any one of claims 1-4, wherein the composition comprises 0.0001 to 100 mg of carotenoid.

6. Composition for use in a method according to any one of claims 1-5, wherein the composition comprises 0.0001 to 25 mg of vitamin K or vitamin K derivative selected from the group consisting of vitamin K1, vitamin K2 and vitamin K3.

7. Composition for use in a method according to any one of claims 1-6, wherein the diseases are collagenoses, such as systemic lupus erythematosus (SLE), polymyositis and dermatomyositis, Sjögren's syndrome, scleroderma, CREST syndrome, Sharp syndrome (mixed connective tissue disorder), Buschke-Ollendorf syndrome or systemic sclerosis.

8. Composition for use in a method according to any one of claims 1-7, wherein the composition is formulated for oral, parenteral or topical administration.

9. Pharmaceutical composition comprising a composition according to any one of claims 1-7 for use in a method according to any one of claims 1-7.

10. Non-therapeutic use of a composition comprising
(a) at least one carotenoid, wherein the carotenoid is lutein, and
(b) at least one vitamin K or vitamin K derivative selected from the group consisting of vitamin K1, vitamin K2 and vitamin K3,
or a dietary food or food supplement comprising such a composition for stabilising or increasing the amount of collagen in human or animal tissue.

11. Non-therapeutic use of claim 10, wherein the human or animal tissue is skin, cartilage or bone.

12. Non-therapeutic use of claim 10 or 11, wherein the composition is formulated for oral, parenteral or topical administration.

## Revendications

1. Composition, comprenant :
(a) au moins un caroténoïde, le caroténoïde consistant en lutéine, et
(b) au moins une vitamine K ou un dérivé de vitamine K, choisis dans l'ensemble constitué par la vitamine K1, la vitamine K2 et la vitamine K3,
destinée à l'utilisation dans un procédé pour le traitement et/ou la prophylaxie d'états ou de maladies qui sont associés à une diminution de la quantité de collagène ou à une dégradation pathologique du collagène.

2. Composition destinée à l'utilisation dans un procédé selon la revendication 1, dans laquelle pour ce qui est de la vitamine K il s'agit de la vitamine K1.

3. Composition destinée à l'utilisation dans un procédé selon la revendication 1, dans laquelle pour ce qui est de la vitamine K il s'agit de la vitamine K2.

4. Composition destinée à l'utilisation dans un procédé selon la revendication 1, dans laquelle pour ce qui est du dérivé de vitamine K il s'agit de la vitamine K3.

5. Composition destinée à l'utilisation dans un procédé selon l'une quelconque des revendications 1-4, dans laquelle la composition comprend de 0,0001 à 100 mg de caroténoïde.

6. Composition destinée à l'utilisation dans un procédé selon l'une quelconque des revendications 1-5, dans laquelle la composition comprend de 0,0001 à 25 mg de vitamine K ou dérivé de vitamine K, choisis dans l'ensemble constitué par la vitamine K1, la vitamine K2 et la vitamine K3.

7. Composition destinée à l'utilisation dans un procédé selon l'une quelconque des revendications 1-6, dans laquelle pour ce qui est des maladies il s'agit de collagénoses, comme par exemple le lupus érythémateux disséminé (LES), la polymyosite et la dermatomyosite, le syndrome de Sjögren, la sclérodermie, le syndrome CREST, le syndrome de Sharp, le syndrome de Buschke-Ollendorff ou la sclérose systémique.

8. Composition destinée à l'utilisation dans un procédé selon l'une quelconque des revendications 1-7, dans laquelle la composition est formulée pour l'administration orale, parentérale ou topique.

9. Composition pharmaceutique, qui comprend une composition selon l'une quelconque des revendications 1-7, destinée à l'utilisation dans un procédé selon l'une quelconque des revendications 1-7.

10. Utilisation non thérapeutique d'une composition comprenant :
(a) au moins un caroténoïde, le caroténoïde consistant en lutéine, et
(b) au moins une vitamine K ou un dérivé de vitamine K, choisis dans l'ensemble constitué par la vitamine K1, la vitamine K2 et la vitamine K3,
ou d'un produit alimentaire diététique ou d'un complément alimentaire, qui comprend une telle composition, pour la stabilisation ou l'augmentation de la quantité de collagène dans un tissu humain ou animal.

11. Utilisation non thérapeutique selon la revendication 10, dans laquelle pour ce qui est du tissu humain ou animal il s'agit de la peau, du cartilage ou de l'os.

12. Utilisation non thérapeutique selon la revendication 10 ou 11, dans laquelle la composition est formulée pour l'administration orale, parentérale ou topique.
